# EUROPEAN PATENT SPECIFICATION

(11) **EP 1 545 982 B1**
(45) Date of publication and mention of the grant of the patent: **15.06.2011**
(21) Application number: 03798638.7
(22) Date of filing: 25.09.2003
(51) Int. Cl.: B65B 69/00, A61G 9/02, A61J 1/10

(54) **DEVICE FOR DISINFECTION OF A BAG CONTAINING BODY FLUID**
VORRICHTUNG ZUR DESINFIZIERUNG EINES KÖRPERFLÜSSIGKEIT ENTHALTENDEN BEUTELS
DISPOSITIF POUR DESINFECTER UNE POCHE RENFERMANT UN FLUIDE ANATOMIQUE

(30) Priority: 26.09.2002 SE 0202839
(43) Date of publication of application: 29.06.2005
(73) Proprietor: Getinge Disinfection AB, 351 15 Växjö (SE)
(72) Inventor: HENDIN, Henrik, S-261 53 Landskrona (SE); LAGERKVIST, Carl-Johan, Getinge Disinfection AB, S-351 15 Växjö (SE); ROESBERG, Mikael, S-269 33 B stad (SE)
(74) Representative: Fritsche, Daniel
(86) International application number: PCT/SE2003/001495
(87) International publication number: WO 2004/028907

(56) References cited:
- EP-A1- 1 010 623
- WO-A1-02/074632
- DE-U1- 29 705 265
- FR-A1- 2 765 553

## Description

### Field of the Invention

This invention relates to a device for use in disinfection of a bag containing body fluid, the device comprising a bag-carrying element and an element for making a hole in the bag for the purpose of removing the body fluid from the same.

### Background Art

In nursing care for instance, use is presently made of a plurality of different bag-like elements for storing, holding and distributing various body fluids. Mention can be made of urine bags for holding urine from a patient, and blood bags which are used, for instance, in blood transfusions for storing and distributing blood to a needy patient. This type of bag is usually disposable and should thus be thrown away after use. Since these bags contain body fluids, or rests of body fluids, the discarded bags must be handled as high-risk material since body fluids may cause contamination and the like of the staff handling the hazardous waste. This special handling and discarding is costly and should therefore be avoided.

A number of devices, above all for handling urine bags, have therefore been developed for the purpose of emptying and disinfecting this type of bags, after which the bag itself is to be considered non-contaminated and thus can be thrown away together with other waste, without requiring special handling. Examples of such devices for handling of urine bags are described, for instance, in US-3,809,577, US-5,190,429, EP-1 010 623 and WO 83/02722. All these publications describe devices with a disinfection chamber, in which a urine bag is placed with its opening directed upwards and its bottom directed downwards in a rack or the like, whereupon a scissor-like object is used to cut a hole in the bottom of the bag, allowing urine to flow out and be removed through an outlet. Subsequently the bags are disinfected by means of a plurality of nozzles or the like located in the chamber. However, these constructions are disadvantageous since merely the outside of the bag can be disinfected in a reliable way. EP-1 010 623, however, describes a device where a disinfection connection is also connected to the inlet of the urine bag, thus allowing also the inside of the bag to be disinfected in a more satisfactory manner. Owing to this construction, however, the person manually operating the device must connect the disinfection connection to the inlet, which is time-consuming work and may result in contamination. An alternative device for disinfection of bags containing body fluid is therefore desirable.

### Summary of the Invention

An object of this invention thus is provide a device for disinfecting, for instance, blood bags, said device causing a reduced risk of contamination and ensuring a high degree of disinfection. Another object of the invention is to provide a device which is flexible. It is also an object of the invention to provide a device which does not suffer from the above drawbacks of prior-art technique.

According to the invention, these and other objects are at least partly achieved by a device of the type as defined in the preamble, further characterised in that the bag-carrying element allows the hole-making element access to the underside of the bag at a hole-making place, the bag-carrying element having an effective supporting surface sloping to the hole-making place, and the device further comprising a means for injecting a cleaning and/or disinfecting fluid, such as one or more of water, vapour or some other disinfectant, said means being adapted to be inserted into the bag on the upper side thereof. Consequently a hole can be made in the bag on the long side thereof which usually is completely without joints and the like, and thus a clean cut can be obtained, which provides for good drainage of body fluid from the bag. This is additionally facilitated by the bag being placed on a sloping supporting surface which slopes to the position in which the opening in the bag for emptying will be made. Thus good emptying of the bag can be ensured. Furthermore the edges of the opening will, owing to material properties and pressure, achieve a slight downward deflection, which additionally simplifies drainage. In prior-art devices, in which a hole is made in a lower edge of the bag, the above phenomenon is prevented owing to the fact that the bag, in a hanging position, tries to maintain its shape.

Preferably, the bag-carrying element comprises an opening to allow access to the underside of the bag, the bag-carrying element having an effective supporting surface sloping to the opening. The opening conveniently allows the hole-making element access essentially from the underside of the bag-carrying element.

Preferably, said opening is an elongate opening, and said hole-making element comprises a knife-shaped element, which is adapted to be inserted along said elongate opening. This contributes to making it possible to control the length of the opening, and thus the emptying rate of the bag, in a simple manner. It is also possible to design the supporting surface so as to provide adequate support for the bag on the sides of the elongate opening. The knife-shaped element is suitably pivotally mounted about a pivot pin which is located under said bag-carrying element. Furthermore a pressure cylinder is suitably arranged to control the pivoting motion of the knife-shaped element about said pin, thus allowing fully automatic hole making.

The device preferably also comprises a means for injecting fluid, such as one or more of water, vapour or some other disinfectant, said means being adapted to be inserted through the side wall of the bag on the upper side for injection of fluid into said bag for disinfection of the same. This allows simple and automatic disinfection of the inside of the bag by rinsing or filling the bag with a desirable agent. It should be noted that it is also possible to insert the means for injection of fluid through the lid of the bag, preferably through the rim of the lid. Since the means is adapted to be inserted directly through the side wall of the bag, and not, for instance, be manually attached to an inlet of the bag, the risk of contamination is reduced significantly. Suitably the means for injection of fluid comprises an injection tube, which is adapted to be inserted through the side wall of the bag on the upper side of the bag. Since the opening in the bag is intended to be arranged in the lower side wall of the bag, a good throughput in the bag will be obtained. Moreover the injection tube is suitably arranged on a yoke element, which is adapted to be lowered over the bag-carrying element in such a manner that the injection tube in the lowered position of the yoke is adapted to be inserted through the wall of the bag, which simplifies the insertion of said injection tube into the bag element. It should also be noted that the device can be used for disinfection of bags with different diameters of the lid, since the yoke construction above can easily be moved to the desired position, in which the injection tube is inserted through the rim of the lid, essentially independently of the diameter of the lid.

It is preferred for the effective supporting surface of the bag-carrying element to slope from its outer edge to the centre of the bag-carrying element. Further said opening is suitably centred in the lower part of the bag-carrying element.

According to one embodiment, the bag-carrying element has the form of a basket.

A portion of the supporting surface of said bag-carrying element is preferably arranged so that a variable inclination to said opening can be obtained. According to an embodiment of the invention, a portion of the supporting surface of said bag-carrying element is spring-loaded by means of a spring element in such a manner that a load-dependent variation of the inclination to said opening is obtained. It is thus possible to obtain a greater inclination to the opening as the bag is being emptied, which allows additionally improved emptying of the bag.

Finally, the device can be in the form of a detachable insert for a disinfecting or sterilising apparatus, which makes it possible to completely remove the device from the disinfecting apparatus if this is to be used for another purpose, which results in a greater useful volume of the chamber in these situations and increased flexibility.

According to a second aspect of the invention, a method is provided, in which a bag containing body fluid can be disinfected by means of a device comprising a bag-carrying element, and an element for making a hole in said bag, said device being intended to be arranged in a disinfection chamber in a disinfecting apparatus. The method comprises arranging the bag, which is to be disinfected, lying on its extended side in the bag-carrying element, inserting a means for injecting cleaning and/or disinfecting fluid into said bag on the upper side of the bag, and inserting the element (8) for making a hole through said bag on the underside of the bag, to make an opening for emptying the bag.

The means for injecting fluid is preferably inserted on the upper side of the bag by a yoke element being lowered over the bag-carrying element from a first position to a second position. Moreover said means is suitably inserted through the side wall of the bag on the upper side. It is also possible to insert said means through the rim of the lid on the bag.

Preferably, the hole-making element is moved from a first to a second position to make said opening for emptying the bag. Furthermore, an effective supporting surface of the bag-carrying element suitably slopes to the opening that has been made. Preferably, the inclination of a portion of the bag-carrying element can be varied to increase the inclination of the bag-carrying element to said opening as the bag is being emptied of its contents.

According to one embodiment, the disinfection chamber is closed before the disinfection of the bag is begun. During the disinfecting operation, the bag is emptied of its contents and cleaning and/or disinfecting fluid is injected into the bag with the aid of the means for injection of fluid.

Making a hole in the bag and injecting fluid into the bag can be carried out in various ways. For the purpose of making a hole in the bag, the hole-making element is preferably inserted through an opening in the bag-carrying element. The hole-making element is conveniently inserted centred in the lower part of the bag-carrying element.

In one embodiment, the bag is filled wholly or partly with cleaning and/or disinfecting fluid before making the opening for emptying, but after inserting the means for injection of fluid. In this embodiment, it is possible to let an adjustable time pass between injection of cleaning or disinfecting fluid and making the opening in the bag. It should be noted that it is also possible to supply cleaning or disinfecting fluid after making the opening in the bag to achieve the required disinfection.

According to another embodiment, body fluid is emptied from the bag wholly or partly before injecting disinfecting fluid, but after making the opening for emptying.

It is thus possible to choose whether to inject fluid into the bag first and then make a hole, or whether to make the hole first and then inject the fluid. It goes without saying that it is also possible to make the hole and inject fluid at the same time. Moreover it is thus possible to fill the bag with fluid and then to let the fluid act for an adjustable time before a hole is made in the bag. All these options contribute to efficient and reliable disinfection of bags containing body fluid by adaptations from case to case.

### Brief Description of the Drawings

This invention will now be described in more detail by way of a currently preferred embodiment and with reference to the accompanying drawing.

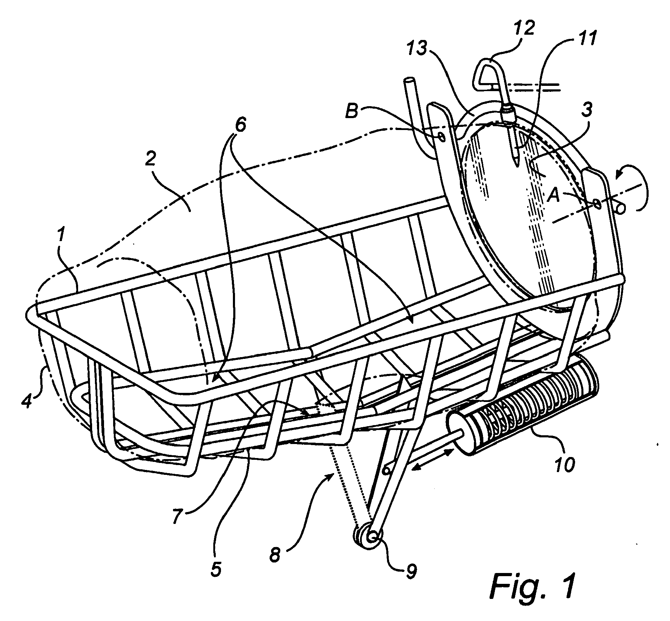

Fig. 1 is a perspective view of a device according to an embodiment of the invention.

### Description of Preferred Embodiments

An embodiment of the invention is shown in Fig. 1. The device shown in Fig. 1 is intended to be placed in a disinfection chamber in a disinfecting apparatus of prior-art type. The device can, as shown in Fig. 1, either be fixedly mounted in the chamber, or be mounted on a rack which is detachably placed in the disinfection chamber for use. The device is specifically adapted for disinfection of blood bags or the like, preferably bags having a substantially circular lid-shaped element of e.g. rigid plastic and comprising inlets and/or outlets for body fluid, the lid-shaped element being fixed to a bag element of non-rigid plastic.

The device comprises a bag-carrying element 1, in this case a basket-shaped element, in which a bag 2 is intended to be laid with its extended side 5 downwards in such a manner that a lid element 3 rests on a lid holding element 15 arranged at one lateral edge of the bag-carrying element 1. The bag-carrying element 1 further has a supporting surface 6 on which the bag element is intended to rest. The supporting surface consists of one or more inclined planes which all slope to an opening 7 arranged in the lower part of the bag-carrying element. In the embodiment shown in Fig. 1, the supporting surface 6 slopes from its outer edges inwards to the centre of the bag-carrying element 1. The opening 7 is centred in the lower part of the bag-carrying element 1, in this case a slot-like opening extending in the longitudinal direction of the bag-carrying element 1. The opening is configured so that a hole-making element 8 can be inserted therethrough from the underside of the bag-carrying element 1 for the purpose of making a hole in a bag placed in the bag-carrying element. In the embodiment in Fig. 1, the hole-making element is a knife 8 which is pivotable about a pivot pin 9 in such a manner that when pivoting the knife between a first and a second outer pivoting position, the knife is inserted through said slot-like opening so that the edge of the knife is passed through the area of the bag-carrying element 1 in which a bag 2 is positioned when it is to be disinfected. Moreover the pivotable knife 8 is via a link connected to an essentially reciprocating element, in this case a pressure cylinder, which is adapted to move the knife 8 between the first and the second outer pivoting position, which provides a simple way of automatically making a hole in the bag element.

As described above, the bag is intended to lie in the bag-carrying element 1 on the extended side wall 5 of the bag, so that the lid element 3 rests on a lid holding element 15 arranged at one lateral edge of the bag-carrying element 1. In the embodiment of the invention as shown in Fig. 1, the lid holding element 15 is a U-shaped element, and a yoke 13 is fixed to one of the legs of the U-shaped element in such a manner that the yoke is pivotable about an axis A in a plane which in this case essentially coincides with the plane defined by the lid holding element 15. A stud B is arranged on the other leg of U-shaped lid holding element, onto which stud the yoke 13, or more particularly a snap portion thereof, can be snapped for the purpose of obtaining stable attachment of the yoke in a position of use. The yoke 13 is thus pivotable about the axis A between a first position where the yoke releases the opening of the U-shaped lid holding element, thus allowing a bag 2 to be placed in the bag-carrying element 1, and a second position in which the yoke 13 is extended between the legs and thus closes the lid holding element, the yoke 13 being fixed in its position by the above-described snap function and, together with the lid holding element 15 grasping the lid of the bag that is to be disinfected. It is possible to exclude the above snap function and let the yoke have a variable position in the grasping position for the purpose of allowing disinfection of bags with different lid diameters and yet providing stable attachment.

A means for injecting disinfecting fluid is arranged on the yoke 13, in this case an injection tube 11 which is connected to a source of disinfecting fluid by connecting means 12. The injection tube is arranged to be inserted through the material of the lid 3 when the yoke 13 is moved from the first to the second position, as described above. Thus, the injection tube can be used to inject disinfecting fluid into the bag. It should also be noted that the injection tube 11 is not exclusively intended to be inserted through the material of the lid 3 of the bag, but it may also be arranged to be inserted into the bag element, preferably on the upper side of the side wall 5 of the bag element.

By the hole-making element 8 being inserted through the underside of the bag and the means 11 for supplying fluid being inserted through the upper side of the bag, a fluid flow path is provided, extending through the internal volume of the entire bag. As a result, efficient and reliable disinfection of the inside of the bag is made possible. Another advantage, achieved by inserting the means 11 for supplying fluid through the lid or side wall of the bag, is that one and the same means 11 can be used for different sizes and variants of bags.

When using the device, a user places a bag 2 which is to be disinfected in the bag-carrying element 1 of the device according to the invention, which as described above is arranged in a disinfection chamber in a disinfecting apparatus (not shown). The user places the bag 2 so that the rim of its lid 3 rests on said lid holding element and the extended side wall 5 of the bag 2 rests on the supporting surface 6 of the bag-carrying element. Subsequently the user pivots the yoke 13 from its first position to its second position of use (as illustrated in Fig. 1) and thereby inserts the injection tube 11 through the rim of the lid 3 in such a manner that the injection opening of the needle is located in the bag 2. Then the user closes the disinfection chamber and starts a disinfection process. In this case, disinfection is begun by a control means sending a signal to the above-described pressure cylinder 10 which in turn moves the knife 8 from the first to the second position, as described above, thereby making an opening or slot in the side wall 5 of the bag, after which the bag 2 is emptied of its contents, which are removed through an outlet in the disinfection chamber. Because the supporting surface 6 is made up of sloping planes sloping to the opening 7, good emptying of the bag is achieved. Then a suitable disinfecting fluid, such as water, vapour or chemical detergents, is supplied to the bag 2 through the injection tube 11, and remaining and used disinfectant is removed through the opening cut in the side wall 5 of the bag. This continues until the desired degree of disinfection has been achieved. At the same time the outside of the bag can be disinfected in prior-art manner by means of a disinfectant distributed through nozzles and the like inside the disinfection chamber. When the desired degree of disinfection has been achieved, the apparatus is stopped, and a user can open the door of the chamber and remove the bag from the bag-carrying element. Owing to the total disinfection that has been performed, the bag can now be thrown away as ordinary refuse and need not be handled as hazardous waste.

As an alternative to that described above, it is also possible first to supply a suitable disinfecting fluid, such as water, vapour or chemical detergents in a suitable amount to the bag 2 containing body fluid. After that the control means sends a signal to the above-described pressure cylinder 10, which in turn moves the knife 8 from the first to the second position as described above, thereby making an opening or slot in the side wall 5 of the bag, after which the bag 2 is emptied of its contents, which are removed through an outlet in the disinfection chamber. The supply of disinfecting fluid may continue to the opened bag until the desired degree of disinfection is achieved. The order of making a hole in the bag and injecting disinfecting fluid can thus be chosen optionally, as required. This also makes it possible to inject disinfecting fluid into the bag and then let the fluid act for a while and, after that, cut the bag open to empty its contents.

Many alternative embodiments of the this invention are feasible without departing from the original inventive idea as defined by the appended claims.

For instance, it should be mentioned that the above-described hole making and emptying of the bag can take place before the actual disinfecting process is begun. Furthermore the insertion of the injection tube can be made automatically by automatic operation of the yoke or the like. The above-described yoke can also be replaced by, for instance, a yoke which is lowered over the device in its longitudinal direction, or the like. Alternatively, the injection tube can be inserted manually through the outer wall of the bag.

According to an alternative embodiment of this invention, the bag-carrying element 1, and thus the supporting surface 6, is divided into two interconnected parts, at least one being spring-loaded by means of a spring element in such a manner that a load-dependent variation of the inclination to the opening 7 is obtained. This makes quicker emptying possible owing to the fact that the inclination of the supporting surface to the opening increases as the bag is being emptied.

It should also be noted that an advantage of this embodiment is that the size of the opening made by the knife-shaped element can easily be adjusted to requirements by adjusting the length of the opening 7 and the size and position of the knife and its pivot pin. Within the scope of the invention, it is also possible to replace the knife with an alternative hole-making element and, correspondingly, also change the shape of the opening 7 in the bag-carrying element and thus achieve other emptying properties. The hole-making place can also be provided in some other way, for instance by a hole-making element, which is retractable into the bag-carrying element, being extended to make a hole in the bag.

Finally it should be appreciated that alternatives to the above-described pressure cylinder can be used to obtain the desired motion of the hole-making element, such as a mechanical construction connected to a motor, or the like.

## Claims

1. A device for use in disinfection of a bag containing body fluid, the device comprising a bag-carrying element (1) and an element (8) for making a hole in the bag for the purpose of removing the body fluid from the same, the bag-carrying element (1) being adapted to carry a bag (2) lying on its extended side (5), **characterised in that** the bag-carrying element (1) allows the hole-making element (8) access to the underside of the bag at a hole-making place, the bag-carrying element (1) having an effective supporting surface (6) sloping to the hole-making place, and the device further comprising a means (11) for injecting cleaning and/or disinfecting fluid, such as one or more of water, vapour or some other disinfectant, said means (11) being adapted to be inserted into the bag on the upper side thereof.

2. A device as claimed in claim 1, wherein the bag-carrying element (1) allows access to the underside of the bag by having an opening (7), the bag-carrying element (1) having an effective supporting surface (6) sloping to the opening (7).

3. A device as claimed in claim 2, wherein said opening allows the hole-making element (8) access essentially from the underside of the bag-carrying element (1).

4. A device as claimed in claim 2 or 3, wherein said opening (7) is an elongate opening, and said hole-making element (8) comprises a knife-shaped element (8), which is adapted to be inserted along said elongate opening (7).

5. A device as claimed in claim 4, wherein said knife-shaped element (8) is pivotally arranged about a pivot pin (9) which is arranged under said bag-carrying element (1).

6. A device as claimed in claim 5, wherein a pressure cylinder (10) is arranged to control the pivoting motion of the knife-shaped element (8) about said pivot pin (9).

7. A device as claimed in any one of the preceding claims, wherein said means (11) for injecting fluid is arranged to be inserted through the side wall (5) of the bag on the upper side.

8. A device as claimed in any one of claims 1-6,
wherein said means (11) for injecting fluid is arranged to be inserted into the bag through the rim of a lid (3).

9. A device as claimed in any one of the preceding claims, wherein said means (11) for injecting fluid comprises an injection tube for injecting fluid into said bag for disinfection thereof.

10. A device as claimed in claim 9, wherein said injection tube (11) is arranged on a yoke element (13) which is adapted to be lowered over the bag-carrying element (1) in such a manner that the injection tube (11) in the lowered position of the yoke element is arranged to be inserted into the bag.

11. A device as claimed in any one of the preceding claims, wherein the effective supporting surface (6) slopes from its outer edges towards the centre of the bag-carrying element (1).

12. A device as claimed in any one of claims 2-11, wherein the opening (7) is centred in the lower part of the bag-carrying element (1).

13. A device as claimed in any one of the preceding claims, wherein the bag-carrying element (1) has the form of a basket.

14. A device as claimed in any one of claims 2-13, wherein a portion of the supporting surface of said bag-carrying element (1) is arranged so that a variable inclination towards said opening (7) can be obtained.

15. A device as claimed in claim 14, wherein said portion of the supporting surface of said bag-carrying element (1) is spring-loaded by means of a spring element in such a manner that a load-dependent variation of the inclination towards said opening is obtained.

16. A device as claimed in any one of the preceding claims, wherein the device is in the form of a detachable insert for a disinfecting or sterilising apparatus.

17. A method for disinfecting a bag (2) containing body fluid by means of a device comprising a bag-carrying element (1) and an element (8) for making a hole in said bag, which device is intended to be arranged in a disinfection chamber in a disinfecting apparatus, comprising
arranging the bag, which is to be disinfected, lying on its extended side (5) in the bag-carrying element (1), inserting a means (11) for injecting cleaning and/or disinfecting fluid into said bag on the upper side of the bag, and inserting the element (8) for making a hole through said bag on the underside of the bag to make an opening for emptying the bag.

18. A method as claimed in claim 17, comprising lowering a yoke element (13) over the bag-carrying element (1) from a first position to a second position, whereby the means for injecting fluid is inserted on the upper side of the bag.

19. A method as claimed in claim 17 or 18, comprising inserting said means (11) for injecting fluid, when being inserted through the bag, through the side wall (5) of the bag on the upper side.

20. A method as claimed in claim 17 or 18, comprising inserting said means (11) for injecting fluid, when inserted through the bag, into the bag through the rim of a lid (3).

21. A method as claimed in any one of claims 17-20, comprising moving the hole-making element (8) from a first position to a second position, thereby making said opening for emptying the bag.

22. A method as claimed in any one of claims 17-21, wherein an effective supporting surface (6) of the bag-carrying element (1) slopes to the opening made in the bag.

23. A method as claimed in any one of claims 17-22, comprising varying the inclination of at least a portion of the bag-carrying element (1) for increasing the inclination to said opening in the bag.

24. A method as claimed in any one of claims 17-23, comprising closing of the disinfection chamber.

25. A method as claimed in any one of claims 17-24, comprising emptying the bag of its contents.

26. A method as claimed in any one of claims 17-25, comprising injecting cleaning and/or disinfecting fluid into the bag with the aid of the means (11) for injecting fluid.

27. A method as claimed in any one of claims 17-26, comprising inserting the hole-making element (8) through an opening (7) in the bag-carrying element (1).

28. A method as claimed in claim 27, comprising inserting the hole-making element (8) centred in the lower part of the bag-carrying element (1).

29. A method as claimed in any one of claims 17-28, comprising filling the bag wholly or partly with cleaning of disinfecting fluid before making the opening for emptying, but after inserting the means (11) for injecting fluid.

30. A method as claimed in claim 29, comprising letting an adjustable time pass between injection of the cleaning or disinfecting fluid and making the opening in the bag.

31. A method as claimed in claim 30, comprising supplying cleaning and/or disinfecting fluid after making the opening in the bag in order to achieve the required disinfection.

32. A method as claimed in any one of claims 17-28, comprising emptying the bag wholly or partly of the body fluid before injecting disinfecting fluid but after making the opening for emptying.

## Patentansprüche

1. Vorrichtung zur Verwendung bei der Desinfektion eines Beutels, der Körperflüssigkeit enthält, wobei die Vorrichtung ein Beutelhalteelement (1) und ein Element (8) zum Herstellen eines Loches in dem Beutel zum Zwecke der Entfernung der Körperflüssigkeit daraus umfasst, wobei das Beutelhalteelement (1) geeignet ist, einen Beutel (2) zu halten, der auf seiner verlängerten Seite (5) liegt, **dadurch gekennzeichnet, dass** das Beutelhalteelement (1) den Zugang des Lochherstellungselements (8) zur Unterseite des Beutels an einer Lochherstellungsstelle ermöglicht, wobei das Beutelhalteelement (1) eine effektive Stützoberfläche (6) aufweist, die zu der Lochherstellungsstelle geneigt ist, und wobei die Vorrichtung ferner ein Mittel (11) zum Injizieren von Reinigungs- und/oder Desinfektionsflüssigkeit wie einem oder mehreren von Wasser, Dampf oder einem anderen Desinfektionsmittel umfasst, wobei das Mittel (11) geeignet ist, um in den Beutel an der Oberseite davon eingesetzt zu werden.

2. Vorrichtung nach Anspruch 1, wobei das Beutelhalteelement (1) den Zugang zu der Unterseite des Beutels **dadurch** ermöglicht, dass es eine Öffnung (7) aufweist, wobei das Beutelhalteelement (1) eine effektive Stützoberfläche (6) aufweist, die zu der Öffnung (7) geneigt ist.

3. Vorrichtung nach Anspruch 2, wobei die Öffnung den Zugang des Lochherstellungselements (8) im Wesentlichen von der Unterseite des Beutelhalteelements (1) ermöglicht.

4. Vorrichtung nach Anspruch 2 oder 3, wobei die Öffnung (7) eine längliche Öffnung ist und das Lochherstellungselement (8) ein messerförmiges Element (8) umfasst, das geeignet ist, um entlang der länglichen Öffnung (7) eingesetzt zu werden.

5. Vorrichtung nach Anspruch 4, wobei das messerförmige Element (8) drehbar um einen Drehzapfen (9) unter dem Beutelhalteelement (1) angeordnet ist.

6. Vorrichtung nach Anspruch 5, wobei ein Druckzylinder (10) angeordnet ist, um die Drehbewegung des messerförmigen Elements (8) um den Drehzapfen (9) zu steuern.

7. Vorrichtung nach einem der vorherigen Ansprüche, wobei das Mittel (11) zum Injizieren von Flüssigkeit angeordnet ist, um durch die Seitenwand (5) des Beutels an der Oberseite eingesetzt zu werden.

8. Vorrichtung nach einem der Ansprüche 1 bis 6, wobei das Mittel (11) zum Injizieren von Flüssigkeit angeordnet ist, um in den Beutel durch den Rand eines Deckels (3) eingesetzt zu werden.

9. Vorrichtung nach einem der vorherigen Ansprüche, wobei das Mittel (11) zum Injizieren von Flüssigkeit einen Injektionsschlauch zum Injizieren von Flüssigkeit in den Beutel vor der Desinfektion umfasst.

10. Vorrichtung nach Anspruch 9, wobei der Injektionsschlauch (11) auf einem Jochelement (13) angeordnet ist, das geeignet ist, um über dem Beutelhalteelement (1) derart abgesenkt zu werden, dass der Injektionsschlauch (11) in der abgesenkten Position des Jochelements in den Beutel eingesetzt werden kann.

11. Vorrichtung nach einem der vorherigen Ansprüche, wobei sich die effektive Stützoberfläche (6) von ihrer äußeren Kante zum Zentrum des Beutelhalteelements (1) neigt.

12. Vorrichtung nach einem der Ansprüche 2 bis 11, wobei die Öffnung (7) in dem unteren Teil des Beutelhalteelements (1) zentriert ist.

13. Vorrichtung nach einem der vorherigen Ansprüche, wobei das Beutelhalteelement (1) die Form eines Korbs aufweist.

14. Vorrichtung nach einem der Ansprüche 2 bis 13, wobei ein Abschnitt der Stützoberfläche des Beutelhalteelements (1) derart angeordnet ist, dass eine variable Neigung zu der Öffnung (7) erhalten werden kann.

15. Vorrichtung nach Anspruch 14, wobei der Abschnitt der Stützoberfläche des Beutelhalteelements (1) mittels eines Federelements derart federbelastet ist, dass eine lastabhängige Variation der Neigung zu der Öffnung erreicht wird.

16. Vorrichtung nach einem der vorherigen Ansprüche, wobei die Vorrichtung in Form eines abnehmbaren Einsatzes für eine Desinfektions- oder Sterilisationsvorrichtung vorliegt.

17. Verfahren zum Desinfizieren eines Beutels (2), der Körperflüssigkeit enthält, mittels einer Vorrichtung, die ein Beutelhalteelement (1) und ein Element (8) zum Herstellen eines Loches in dem Beutel umfasst, wobei die Vorrichtung zur Anordnung in einer Desinfektionskammer in einem Desinfektionsgerät bestimmt ist, umfassend
Anordnen des zu desinfizierenden Beutels, der auf seiner verlängerten Seite (5) in dem Beutelhalteelement (1) liegt, Einsetzen eines Mittels (11) zum Injizieren einer Reinigungs- und oder Desinfektionsflüssigkeit in den Beutel an der Oberseite des Beutels und Einsetzen des Elements (8) zur Herstellung eines Loches durch den Beutel an der Unterseite des Beutels, um eine Öffnung zum Entleeren des Beutels herzustellen.

18. Verfahren nach Anspruch 17, umfassend das Absenken eines Jochelements (13) über dem Beutelhalteelement (1) von einer ersten Position in eine zweite Position, wodurch das Mittel zum Injizieren von Flüssigkeit an der Oberseite des Beutels eingesetzt wird.

19. Verfahren nach Anspruch 17 oder 18, umfassend, während des Einsetzens durch den Beutel, das Einsetzen des Mittels (11) zum Injizieren von Flüssigkeit durch die Seitenwand (5) des Beutels an der Oberseite.

20. Verfahren nach Anspruch 17 oder 18, umfassend, während des Einsetzens durch den Beutel, das Einsetzen des Mittels (11) zum Injizieren von Flüssigkeit durch den Rand eines Deckels (3).

21. Verfahren nach einem der Ansprüche 17 bis 20, umfassend das Bewegen des Lochherstellungselements (8) von einer ersten Position in eine zweite Position, wodurch die Öffnung zum Entleeren des Beutels hergestellt wird.

22. Verfahren nach einem der Ansprüche 17 bis 21, wobei sich eine effektive Stützoberfläche (6) des Beutelhalteelements (1) zu der in dem Beutel hergestellten Öffnung neigt.

23. Verfahren nach einem der Ansprüche 17 bis 22, umfassend das Variieren der Neigung mindestens eines Abschnitts des Beutelhalteelements (1) zum Erhöhen der Neigung zu der Öffnung in dem Beutel.

24. Verfahren nach einem der Ansprüche 17 bis 23, umfassend das Verschließen der Desinfektionskammer.

25. Verfahren nach einem der Ansprüche 17 bis 24, umfassend das Entleeren des Beutelinhalts.

26. Verfahren nach einem der Ansprüche 17 bis 25, umfassend das Injizieren von Reinigungs- und/oder Desinfektionsflüssigkeit in den Beutel mit Hilfe des Mittels (11) zum Injizieren von Flüssigkeit.

27. Verfahren nach einem der Ansprüche 17 bis 26, umfassend das Einsetzen des Lochherstellungselements (8) durch eine Öffnung (7) in dem Beutelhalteelement (1).

28. Verfahren nach Anspruch 27, umfassend das Einsetzen des Lochherstellungselements (8), das in dem unteren Teil des Beutelhalteelements (1) zentriert ist.

29. Verfahren nach einem der Ansprüche 17 bis 28, umfassend das vollständige oder teilweise Befüllen des Beutels mit Reinigungs- oder Desinfektionsflüssigkeit vor dem Herstellen der Öffnung zum Entleeren, jedoch nach dem Einsetzen des Mittels (11) zum Injizieren von Flüssigkeit.

30. Verfahren nach Anspruch 29, umfassend das Vergehenlassen eines einstellbaren Zeitraums zwischen der Injektion der Reinigungs- oder Desinfektionsflüssigkeit und dem Herstellen der Öffnung in dem Beutel.

31. Verfahren nach Anspruch 30, umfassend das Zuführen von Reinigungs- und/oder Desinfektionsflüssigkeit nach dem Herstellen der Öffnung in dem Beutel, um die erforderliche Desinfektion zu erzielen.

32. Verfahren nach einem der Ansprüche 17 bis 28, umfassend das vollständige oder teilweise Entleeren der Körperflüssigkeit vor dem Injizieren von Desinfektionsflüssigkeit, jedoch nach dem Herstellen der Öffnung zum Entleeren.

## Revendications

1. Dispositif destiné à être utilisé pour la désinfection d'un sac contenant du fluide physiologique, ce dispositif comprenant un élément porteur de sac (1) et un élément (8) permettant de faire un trou dans le sac afin de retirer le fluide de celui-ci, l'élément porteur de sac (1) étant apte à porter un sac (2) reposant sur son côté déployé (5), **caractérisé en ce que** l'élément porteur de sac (1) permet à l'élément perceur de trou (8) d'accéder à la face inférieure du sac à un endroit de perçage de trou, l'élément porteur de sac (1) ayant une surface support effective (6) descendant vers l'endroit de perçage de trou et le dispositif comprenant en outre un moyen (11) d'injection de fluide nettoyant et/ou désinfectant comme un ou plusieurs fluides parmi l'eau, la vapeur ou un autre désinfectant, ledit moyen (11) étant apte à être inséré dans le sac sur sa face supérieure.

2. Dispositif selon la revendication 1, dans lequel l'élément porteur de sac (1) permet d'accéder à la face inférieure du sac du fait qu'il a un orifice (7), l'élément porteur de sac (1) ayant une surface support effective (6) descendant vers l'orifice (7).

3. Dispositif selon la revendication 2, dans lequel ledit orifice permet à l'élément perceur de trou (8) d'accéder essentiellement depuis la face inférieure de l'élément porteur de sac (1).

4. Dispositif selon la revendication 2 ou 3, dans lequel ledit orifice (7) est un orifice oblong, et ledit élément perceur de trou (8) comprend un élément en forme de couteau (8) qui est apte à être inséré le long dudit orifice oblong (7).

5. Dispositif selon la revendication 4, dans lequel ledit élément en forme de couteau (8) est disposé en pivotement autour d'une broche de pivotement (9) qui est disposée en dessous dudit élément porteur de sac (1).

6. Dispositif selon la revendication 5, dans lequel un cylindre de compression (10) est disposé pour contrôler le mouvement de pivotement de l'élément en forme de couteau (8) autour de ladite broche de pivotement (9).

7. Dispositif selon l'une quelconque des revendications précédentes, dans lequel ledit moyen (11) d'injection de fluide est conçu pour être inséré à travers la cloison latérale (5) du sac sur la face supérieure.

8. Dispositif selon l'une quelconque des revendications 1 à 6, dans lequel ledit moyen (11) d'injection de fluide est conçu pour être inséré dans le sac à travers le rebord d'un couvercle (3).

9. Dispositif selon l'une quelconque des revendications précédentes, dans lequel ledit moyen (11) d'injection de fluide comprend un tube d'injection pour injecter du fluide dans ledit sac pour le désinfecter.

10. Dispositif selon la revendication 9, dans lequel ledit tube d'injection (11) est disposé sur un élément à bascule (13) qui est apte à être abaissé au dessus de l'élément porteur de sac (1) de manière à ce que le tube d'injection (11), en position abaissée de l'élément à bascule, soit dans une position permettant son insertion dans le sac.

11. Dispositif selon l'une quelconque des revendications précédentes, dans lequel la surface support effective (6) descend depuis ses bords extérieurs vers le centre de l'élément porteur de sac (1).

12. Dispositif selon l'une quelconque des revendications 2 à 11, dans lequel l'orifice (7) est centré dans la partie inférieure de l'élément porteur de sac (1).

13. Dispositif selon l'une quelconque des revendications précédentes, dans lequel l'élément porteur de sac (1) a la forme d'un panier.

14. Dispositif selon l'une quelconque des revendications 2 à 13, dans lequel une partie de la surface support dudit élément porteur de sac (1) est conçue de manière à pouvoir obtenir une inclinaison variable vers ledit orifice (7).

15. Dispositif selon la revendication 14, dans lequel ladite section de la surface support dudit élément porteur de sac (1) est chargée par ressort au moyen d'un élément à ressort de manière à obtenir une variation dépendant de la charge de l'inclinaison vers ledit orifice.

16. Dispositif selon l'une quelconque des revendications précédentes, dans lequel le dispositif a la forme d'un insert détachable pour un appareil désinfectant ou de stérilisation.

17. Procédé de désinfection d'un sac (2) contenant du fluide physiologique au moyen d'un dispositif comprenant un élément porteur de sac (1) et un élément (8) permettant de faire un trou dans ledit sac, lequel dispositif est conçu pour être disposé dans une chambre de désinfection d'un appareil désinfectant, comprenant
la disposition du sac à désinfecter, reposant sur son côté déployé (5), dans l'élément porteur de sac (1), l'insertion d'un moyen (11) d'injection de fluide nettoyant et/ou désinfectant et l'insertion de l'élément (8) permettant de faire un trou dans ledit sac sur la face inférieure du sac pour pratiquer un orifice permettant de vider le sac.

18. Procédé selon la revendication 17, comprenant l'abaissement d'un élément à bascule (13) au dessus de l'élément porteur de sac (1) d'une première position vers une seconde position, le moyen d'injection de fluide étant inséré sur la face supérieure du sac.

19. Procédé selon la revendication 17 ou 18, comprenant l'insertion dudit moyen (11) d'injection de fluide, une fois inséré dans le sac, dans la cloison latérale (5) du sac sur la face supérieure.

20. Procédé selon la revendication 17 ou 18, comprenant l'insertion dudit moyen (11) d'injection de fluide, une fois inséré dans le sac, dans le sac à travers le rebord d'un couvercle (3).

21. Procédé selon l'une quelconque des revendications 17 à 20, comprenant le déplacement de l'élément perceur de trou (8) d'une première position vers une seconde position, en pratiquant ainsi ledit orifice permettant de vider le sac.

22. Procédé selon l'une quelconque des revendications 17 à 21, dans lequel une surface support effective (6) de l'élément porteur de sac (1) descend vers l'orifice pratiqué dans le sac.

23. Procédé selon l'une quelconque des revendications 17 à 22, comprenant la variation de l'inclinaison d'au moins une partie de l'élément porteur de sac (1) pour augmenter l'inclinaison vers ledit orifice du sac.

24. Procédé selon l'une quelconque des revendications 17 à 23, comprenant la fermeture de la chambre de désinfection.

25. Procédé selon l'une quelconque des revendications 17 à 24, comprenant le fait de vider le sac de son contenu.

26. Procédé selon l'une quelconque des revendications 17 à 25, comprenant l'injection de fluide nettoyant et/ou désinfectant dans le sac à l'aide du moyen (11) d'injection de fluide.

27. Procédé selon l'une quelconque des revendications 17 à 26, comprenant l'insertion de l'élément perceur de trou (8) à travers un orifice (7) de l'élément porteur de sac (1).

28. Procédé selon la revendication 27, comprenant l'insertion de l'élément perceur de trou (8) centré dans la partie inférieure de l'élément porteur de sac (1).

29. Procédé selon l'une quelconque des revendications 17 à 28, comprenant le remplissage total ou partiel avec du fluide nettoyant ou désinfectant avant de pratiquer le trou de vidange mais après l'insertion du moyen (11) d'injection de fluide.

30. Procédé selon la revendication 29, comprenant le fait de laisser s'écouler un temps ajustable entre l'injection du fluide nettoyant ou désinfectant et de pratiquer l'orifice dans le sac.

31. Procédé selon la revendication 30, comprenant l'apport de fluide nettoyant et/ou désinfectant dans le sac après avoir pratiqué l'orifice dans le sac pour réaliser la désinfection requise.

32. Procédé selon l'une quelconque des revendications 17 à 28, comprenant la vidange totale ou partielle du fluide physiologique avant d'injecter du fluide désinfectant, mais après avoir pratiqué l'orifice de vidange.
